# EUROPEAN PATENT APPLICATION

(11) **EP 4 807 340 A1**
(43) Date of publication of application: **16.09.2026**
(21) Application number: 26159854.4
(22) Date of filing: 20.02.2026
(51) Int. Cl.: G01N 1/28, G01N 33/32, G01N 1/44, G01N 5/04, G01N 17/00, G01N 15/02

(54) **PARTICLE DETECTION METHOD FOR COATING COMPOSITIONS**

(30) Priority: 12.03.2025 EP 25163336
(71) Applicant: BASF SE, 67056 Ludwigshafen am Rhein (DE)
(72) Inventor: Schmidt, Patrizia Marie, 67056 Ludwigshafen am Rhein (DE); Wohlleben, Wendel, 68165 Mannheim (DE); Willerich, Immanuel, 67056 Ludwigshafen am Rhein (DE); Armbrust, Claudia, 67056 Ludwigshafen am Rhein (DE); Gruendling, Till, 67056 Ludwigshafen am Rhein (DE)
(74) Representative: BASF IP Association

(57) **Abstract**

The present invention relates to a particle, especially micro- and nanoplastics, composite and inorganics, sampling and detection method and device for polymer comprising coating compositions, preferably paints, with exposure to UV light and humidity during their life cycle characterized by the following steps:
a. the application and drying of a defined weight of a polymer containing coating formulation on a substrate to form a coating film
b. Ageing of the coating film under (a) using UV light and humidity.
c. Sampling dissolved organic compounds (DOC), inorganics, micro- and nanoplastics by
c1) absolute mass loss of the coating film under (b) by gravimetrical methods.
c2) dissolved organic compounds with immersion in water
c3) inorganic, micro- and nanoplastics with mechanical treatment (e.g. water pressure or ultrasound optionally in the presence of surfactant), and

d. determining the concentration of the released species under c. by quantifying at least one of the following species
d1) dissolved organic carbon,
d2) inorganics, including its fraction in composites,
d3) micro-, nanoplastics, including its fraction in composites,
d4) total number of particles

with analytical detection methods.

## Description

The present invention relates to a particle, especially micro- and nanoplastics, composite and inorganics, sampling and detection method and device for polymer comprising coating compositions, preferably paints, with exposure to UV light and humidity during their life cycle.

Micro- and nanoplastics are small plastic particles, typically less than 5 millimeters in size, that are solid and insoluble in water, and may be unintentionally released due to environmental ageing of plastic products. Micro- and nanoplastic particles contain polymer and may originate from a variety of sources, including the breakdown of larger plastic debris, microbeads in personal care products, synthetic fibers from textiles, and paints. Due to their small size, microplastics can be ingested by a wide range of organisms, from plankton to larger marine animals, leading to potential adverse effects on wildlife and human health.

The formation of micro- and nanoplastics from synthetic polymers is a subject of relevance for regulatory bodies, industry and stakeholders and its pervasive nature in the environment has prompted extensive research into its sources, distribution, and impacts. Due to the potential environmental impact, the development of detection, isolation, purification and quantification methods for micro- and nanoplastic particles is an emergent technical field supporting the goal to understand the formation and release of such particles and to enable the development of new materials with reduced emission profiles.

The present invention addresses these needs by presenting a novel method for the identification and analysis of polymer particles, such as microplastics, offering significant advances in environmental monitoring and protection.

US11913863 describes a pyrolysis device especially suitable for microplastic detection in the field but specifically focusing on technical details of a pyrolysis device.

WO2025/015171 describes an automated spectroscopic device using IR to detect microplastic particles with spatial resolution.

KR20210002420 describes a workflow for the isolation and detection of microplastic particles from seawater, involving a filtration step and the oxidation of organic matter before dissolving the microplastic particles in an organic solvent.

Literature indicates that UV aging is one of the most severe environmental stressors, which causes fragmentation of polymeric materials. This process not only results in the release of fillers, microplastics, and nanoplastics but also leads to the degradation of the solid polymer into CO₂, water, volatile organic compounds, and dissolved organic carbon (DOC) (Ward, C. P., C. J. Armstrong, A. N. Walsh, J. H. Jackson and C. M. Reddy (2019). Environmental Science & Technology Letters 6(11): 669-674; Pfohl, P., M. Wagner, L. Meyer, P. Domercq, A. Praetorius, T. Hüffer, T. Hofmann and W. Wohlleben (2022). Environ Sci Technol 56(16): 11323-11334).

For simulated release studies, a combination of environmental aging and mechanical treatment is needed (Ruggiero, E., K. Vilsmeier, P. Mueller, S. Pulbere and W. Wohlleben (2019). Environmental Science: Nano 6(10): 3039-3048, Reineccius, J., M. Schonke and J. J. Waniek (2023). Environ Sci Technol 57(2): 963-975).

To date, no standardized method exists to investigate the release of polymer particles such as micro- and nanoplastic particles from polymer comprising coatings because of the complexity of the issue, namely the potential release of multicomponent fragments ((in)organic fillers and pigments in a continuous or network-like polymeric binder matrix), as well as water-soluble non-particulate organic material (Diana, Z. T., Y. Chen and C. M. Rochman (2025). Environmental Toxicology and Chemistry 44(1): 26-44).

It is an object of the invention to establish a standardized laboratory workflow to determine the release not only of polymer particles, such as micro- and nanoplastics, but also of inorganics and composites from artificially weathered coating formulations while being able to distinguish small water-soluble non-particulate organics from polymer particles and inorganic species while also determining a maximum threshold for the release of CO₂, water and volatile compounds. Furthermore, combinations of some of the described preparation and analytical techniques for the determination of the different compounds can also be applied to samples collected on naturally weathered objects.

It was surprisingly found, that the objective is achieved by a sampling and particle detection method targeted for micro- and nanoplastics, inorganics and composites for polymer comprising coating compositions, based on the following steps,
a. the application and drying of a defined weight of a polymer containing coating formulation on a substrate to form a coating film
b. Ageing of the coating film using UV light and humidity.
c. Sampling dissolved organic compounds (DOC), inorganics, micro- and nanoplastics by
   c1) absolute mass loss of the coating film under (b) by gravimetrical methods.
   c2) dissolved organic compounds with immersion in water
   c3) inorganic, micro- and nanoplastics with mechanical treatment (e.g. water pressure or ultrasound optionally in the presence of surfactant), and
d. determining the concentration of the released species under c. by quantifying at least one of the following species
   d1) dissolved organic carbon,
   d2) inorganics, including its fraction in composites,
   d3) micro-, nanoplastics, including its fraction in composites,
   d4) total number of particles
with analytical detection methods.

In a preferred embodiment the ageing under (a) is performed according to ISO 4892.

In a preferred embodiment the determination of the concentration of the released species under c. is detected by at least one or a combination of the following analytical detection methods
d1) total organic carbon analyzer,
d2) inductively coupled plasma optical emission spectroscopy (ICP-OES),
d3) pyrolysis GCMS or thermogravimetric analysis or thermoextraction and desorption GCMS,
d4) a particle counting device, a dynamic and static light scattering device or an optical microscopic device.

In a further preferred embodiment the determination of the concentration of the released species under c. is detected by a combination of the following analytical detection methods
d1) total organic carbon analyzer,
d2) inductively coupled plasma optical emission spectroscopy (ICP-OES),
d3) pyrolysis GCMS
d4) a particle counting device.

A further embodiment of the invention is the use of a particle detection device, comprising the following steps,
a. the application and drying of a defined weight of a polymer containing coating formulation on a substrate to form a coating film
b. Ageing of the coating film using UV light and humidity.
c. Sampling dissolved organic compounds (DOC), inorganics, micro- and nano plastics by
   c1) absolute mass loss of the coating film under (b) by gravimetrical methods.
   c2) dissolved organic compounds with immersion in water
   c3) inorganic, micro- and nano plastics with mechanical treatment (e.g. water pressure or ultrasound optionally in the presence of surfactant), and
d. determining the concentration of the released species under c. is detected by at least one or a combination of the following analytical detection methods
   d1) total organic carbon analyzer,
   d2) inductively coupled plasma optical emission spectroscopy (ICP-OES),
   d3) pyrolysis GCMS or thermogravimetric analysis or thermoextraction and desorption GCMS,
   d4) a particle counting device, a dynamic and static light scattering device or an optical microscopic device.

In a preferred embodiment the ageing under (a) is performed according to ISO 4892.

A preferred particle detection device is based on a combination of the following analytical detection methods
d1) total organic carbon analyzer,
d2) inductively coupled plasma optical emission spectroscopy (ICP-OES),
d3) pyrolysis GCMS
d4) a particle counting device.

The detection method can detect particles below 120 µm size down to 20 nm particle diameter, as well as dissolved organic compounds (DOC) < 20 nm.

In the current workflow, large particles above 120 µm require further processing like grinding to avoid high errors due to sample inhomogeneity.

Here and throughout the specification, the term "particle" means micro- and nanoplastics, inorganics and composites and covers all synthetic polymer particles below 5 mm that are generally organic, insoluble (in water) and resist degradation.

Here and throughout the specification, the term "inorganics" or "inorganic particles" means inorganic and organic pigments, fillers, or matting agents.

Organic pigments are non-polymeric particles comprising organic dyes prepared as micro- or nanoscopic particles.

Here and throughout the specification, the term "dissolved organic carbon" means smaller molecules that are too high in molecular weight to evaporate during the treatment under (b) but low enough in molecular weight to pass through a filter with 20 nm pore size.

Here and throughout the specification, the term "composites" means micro- and nanoplastics attached to inorganic particles so that both particles are counted as one particle in a particle counter.

Here and throughout the specification, the term "microplastic" or "nanoplastic" means nanoplastics, microplastics and large microplastic as defined below:
Microplastics may show various shapes. Typically, a large microplastics object represents an item consisting of plastics or a part of an end-user product or a fragment of the respective item and is between 1 mm and 5 mm in size. Microplastic are any solid plastic particle insoluble in water with dimension between 1 µm and 1000 µm (= 1 mm).

Nanoplastics are all plastic particles with sizes below 1 µm as defined in ISO TR 21960: 2021.

The critical aspect of step a is the utilization of an inert substrate, such as glass, for the coating application. While it can be advantageous for the substrate to closely resemble that used in the actual environmental application, using glass may be preferable to minimize the potential influence of impurities from the substrate on the analysis.

The preferable application before the artificial aging is via doctor-blading to achieve a homogeneous coating film. Non-inert substrates can also be used but make it difficult to accurately determine the mass loss and it must be made sure that there is no overlap of matrix signals with the target analytes.

Step b involves a standardized artificial aging protocol for the coated substrates. Any recognized aging protocol, such as ISO 4892, may be employed, provided that no rain events are introduced, as this could result in the loss of released particles.

It is preferable to maintain a high relative humidity during this step, as the presence of water can significantly impact the aging process. It is also possible to investigate samples aged under natural weathering conditions with parts of the analytical workflow described in step c and d. If rain events are applied during artificial weathering, rainwater can be collected and analyzed likewise with parts of step d. The weight of the coated substrates should be determined before and after aging to allow for a precise determination of the weight loss of the coating in absolute mass and relative to the original coat weight.

The sampling protocol outlined in step c consists of a series of actions that progressively apply mechanical treatment to both pristine and aged samples. The aim is to collect only those loosely attached particles according to the definition above that are released from the surface of the paint following artificial or environmental aging, while minimizing flaking induced by the workflow itself and therefore avoiding an overestimation of the release. Flaking in the current context means the release of fractures induced by the workflow rather than the artificial ageing.

Given that the stability of the coatings against flaking is influenced by factors such as coating composition and adhesion, the protocol requires halting at various sampling stages as soon as visual signs of flaking are detected. Before the start of the actual sampling workflow, the coated substrates should be weighed again to determine the mass loss during aging to determine the maximum release of VOC, CO₂ and H₂O. This is only possible with inert substrates, such as glass, which are not capable to take up water during the high humidity aging. For non-inert substrates, equilibration protocols are required that ensure the same humidity level.

In the workflow, the sampling process begins with immersion in water, which is a process with low energy input only triggering solution processes without breaking the adhesion forces between released particles and the coating film. The following treatment by water-spraying under pressure optionally with a diluted surfactant solution using an airbrush pistol simulates intense rain events and facilitates the release of particles according to the definition above from the coating. Then ultrasonication (US) at 20% and finally 100% intensity again optionally with surfactant solution are considered suitable to sample measurable fragments by applying harsh mechanical stress. Both airbrush and ultrasonication steps release micro- and nanoplastics, inorganic particles and composite particles as defined above.

Additionally, it is feasible to employ other forms of mechanical treatment during the sampling process. To evaluate releases that are solely induced by the artificial or environmental aging of the coatings, the protocol should be halted immediately upon the occurrence of flaking, and only the fine fraction of particles smaller than 120 µm should be analyzed. If the assessment of flaking itself is of interest, the same analytical methods can also be utilized to quantify the components present in the flakes > 120 µm after a suitable micronization step.

A combination of analytical techniques, as outlined in step d, can be employed to evaluate the release of various species from coatings. Depending on the specific species of interest, one or more techniques can be utilized.

Dissolved organic compounds (DOC) measurements can only be performed on the shaker samples, unless surfactants are not used during the mechanical treatment steps.

However, it is advisable to incorporate surfactants in these mechanical treatment processes to enhance the release of particles and ensure a homogeneous dispersion of the released material, thus minimizing particle loss.

Particle counting can be performed with an Abakus^{®} particle counter, an analytical device used to measure and quantify the number and size of particles suspended in a fluid, typically air or liquids.

This method utilizes a laser or light source to illuminate particles as they pass through a sensing area, where they scatter light. The scattered light is then detected and analyzed to determine the particle size distribution and concentration.

Total particle counts can be assessed at all sampling stages, that is to determine micro- and nanoplastics, composites and inorganic particles; however, a blank control (treated water in the absence of a coated substrate) must be subtracted from the measured data for each treatment step. It is important to note that the data obtained from the particle counter does not differentiate between the compositions of the various particulate species.

Dynamic Light Scattering (DLS) is a technique used to measure the size distribution of small particles in suspension or polymers in solution. It works by shining a laser beam through a sample, causing the particles to scatter the light. As the particles move due to Brownian motion, the scattered light experiences fluctuations in intensity. These fluctuations are analyzed to determine the diffusion coefficient of the particles, which can then be related to their hydrodynamic diameter using the Stokes-Einstein equation.

Static Light Scattering (SLS) is an analytical technique used to determine the size and molecular weight of macromolecules, colloids, and nanoparticles in solution. Unlike Dynamic Light Scattering (DLS), which measures fluctuations in scattered light over time, SLS measures the average intensity of light scattered by particles at multiple fixed angles. This technique is based on the principle that larger particles scatter light more intensely than smaller ones.

By analyzing the intensity of the scattered light as a function of concentration and angle, SLS can provide information about the particle size distribution and molecular weight.

Small-Angle X-ray Scattering (SAXS) is a powerful analytical technique used to investigate the structure of materials at the nanoscale, typically in the range of 1 to 100 nanometers. SAXS involves directing X-rays at a sample and measuring the intensity of the scattered X-rays at small angles. The scattering pattern provides information about the size, shape, and arrangement of particles, macromolecules, or other structures within the sample.

Small-Angle Neutron Scattering (SANS) is an analytical technique used to study the structure of materials at the nanoscale, similar to Small-Angle X-ray Scattering (SAXS). However, SANS utilizes neutrons instead of X-rays to probe the sample. The technique involves directing a beam of neutrons at a sample and measuring the intensity of the scattered neutrons at small angles.

Optical microscopy devices e.g. Raman and Infrared (IR) microscopy are analytical techniques used to obtain chemical information, particle number and sizes of materials at a microscopic scale.

Raman microscopy utilizes inelastic scattering of monochromatic light, typically from a laser, to provide information about molecular vibrations, which can be related to the chemical composition and structure of a sample. When light interacts with molecular bonds, it can be scattered with a change in energy, which is detected to create a Raman spectrum. This technique is particularly useful for studying organic compounds, biological samples, and materials where traditional methods may fail due to sample opacity.

Infrared microscopy, on the other hand, involves the absorption of infrared light by a sample, which corresponds to the vibrational modes of molecules. When infrared radiation passes through or reflects off a sample, specific wavelengths are absorbed, providing insight into functional groups and molecular structures. IR microscopy is commonly used in the analysis of polymers, biological tissues, and other materials where chemical characterization is needed.

Both techniques are complementary, often used in conjunction to provide a comprehensive understanding of a material's chemical composition and properties at the microscopic level.

Pyrolysis Gas Chromatography-Mass Spectrometry (Py-GC/MS) is an analytical technique used to analyze complex organic materials by thermally decomposing them in the absence of oxygen. During pyrolysis, the sample is heated to high temperatures in an inert gas atmosphere, causing it to break down into smaller volatile compounds. These compounds are then separated using gas chromatography. As the compounds elute from the GC, they enter the mass spectrometer, which detects and identifies them based on their mass-to-charge ratio. This method is particularly useful for characterizing polymers, as it provides detailed information on the chemical composition and structure of the analytes present.

The pure polymer binder and/or the liquid paint formulation can be used for calibration.

Thermal Desorption Gas Chromatography-Mass Spectrometry (TED-GC/MS), is an analytical technique that combines thermal desorption, gas chromatography, and mass spectrometry to analyze volatile and semi-volatile organic compounds. This method is particularly useful for identifying trace levels of contaminants in various matrices, such as air, water, soil, and biological samples. The process begins with thermal desorption, where samples are heated to degrade materials and to release volatile compounds. These compounds are then separated using gas chromatography. As the compounds elute from the GC, they enter the mass spectrometer, which detects and identifies them based on their mass-to-charge ratio. This method is particularly useful for characterizing polymers, as it provides detailed information on the chemical composition and structure of the analytes present.

Thermogravimetric Analysis (TGA) is a thermal analysis technique used to measure the change in mass of a material as it is heated, cooled, or held at a constant temperature. This method provides valuable information about the thermal stability, composition, and decomposition characteristics of materials. During TGA, a sample is subjected to a controlled temperature program, while its mass is monitored continuously.

Inductively Coupled Plasma Optical Emission Spectroscopy (ICP-OES) is an analytical technique used for the quantitative determination of trace elements in various samples. In this method, a sample is introduced into a high-temperature plasma, generated by an inductively coupled radio frequency source. The energy from the plasma excites the atoms in the sample, causing them to emit light at characteristic wavelengths. By measuring the intensity of this emitted light, the concentration of specific elements can be determined.

ICP-OES can be employed to determine the total released inorganics, also applicable in all mechanical treatment steps.

Total Organic Carbon (TOC) analysis is an analytical method used to quantify the amount of organic carbon present in a sample. This technique measures the carbon content in organic compounds, providing insights into water quality, and the evaluation of industrial processes. The TOC analysis typically involves oxidizing the organic carbon to carbon dioxide, which is then measured using methods such as infrared detection or conductivity measurement. After filtration with a filter with a mesh size < 20 nm only dissolved organic carbon (DOC) is detected by the TOC analyzer. DOC can be formed during artificial aging and is released into water.

The inventive workflow is associated with several benefits:
The workflow comprises a defined coating film preparation, ageing, release and analysis methods under conditions that allow to determine the mass of all particles released as well as the sum of dissolved organic materials.

The established protocol ensures the effective collection of the particles, such as micro- and nanoplastics and is designed to differentiate between particles released as a result of environmental or artificial aging and those released due to flaking caused by high mechanical stress. This distinction helps to prevent any overestimation of microplastic release.

Here and throughout the specification, the term "coating" or "coating composition" means any coating composition, comprising solvent-borne alkyds, or aqueous emulsion polymers containing water or solvent as the continuous phase in an amount sufficient to achieve flowability, e.g. paints, adhesives, sealants, or polymer modified plasters or renders, insulation coatings, corrosion protection coatings, water-proofing coatings and vibration dampening coatings.

In a preferred embodiment of the invention the coating compositions are paints.

Also, pure polymer compositions can be investigated in the same way, e.g. pressure sensitive adhesives or clear coats.

The coating compositions contains a "binder polymer" in the form of an aqueous or solvent-borne polymer, preferably an aqueous polymer latex; and at least one further ingredient, which is conventionally used in coating compositions which is not a binder, such as pigments or fillers, hereinafter also referred to as "inorganics".

The invention uses as binder polymer, emulsion polymers which comprises, in copolymerized form, esters of acrylic and/or methacrylic acid with alkanols having from 1 to 12 carbon atoms, and/or styrene, or of styrene and/or butadiene, or of vinyl chloride and/or vinylidene chloride, of vinyl acetate, vinyl propionate, vinyl esters of versatic acid, vinyl esters of long-chain fatty acids, and/or ethylene, present in aqueous dispersion.

But in principle all binders in the coating sector can be used, e.g. water- or solvent-borne alkyd resins, powder coatings.

Solvent-borne coatings, as alkyd resins and their process for preparing are familiar to the person skilled in the art. The procedure has been extensively described in the prior art (see WO2008/152078; US 6,333,378, DE3132937).

Alkyd resins are derived from polyols and organic acids, including dicarboxylic acids or carboxylic acid anhydrides, and triglyceride oils (U.Poth, Polyester and alkyd resins, Vincentz Network 2005).

They are commercially available, e.g. with trademarks WorléeKyd ^{®}AC 2550 , WorléeKyd^{®} B 4901, WorléeKyd ^{®}B 865, WorléeKyd ^{®}RL 1290 from Worlée or Synthalat^{®} from Synthopol.

Emulsion polymers are familiar to the person skilled in the art and are produced by way of example in the form of an aqueous polymer dispersion via free-radical-initiated aqueous emulsion polymerization of ethylenically unsaturated monomers. The procedure for preparing emulsion polymers in an aqueous medium has been extensively described and is therefore sufficiently familiar to the skilled person [cf. in this regard Emulsion Polymerization in Encyclopedia of Polymer Science and Engineering, vol. 8, pages 659 ff. (1987); D.C. Blackley, in High Polymer Latices, vol. 1, pages 35 ff. (1966); H. Warson, The Applications of Synthetic Resin Emulsions, chapter 5, pages 246 ff. (1972); D. Diederich, Chemie in unserer Zeit 24, pages 135 to 142 (1990); Emulsion Polymerisation, Interscience Publishers, New York (1965); DE-A 40 03 422; and Dispersionen synthetischer Hochpolymerer, F. Hölscher, Springer-Verlag, Berlin (1969)]. Typical procedures for aqueous emulsion polymerization of ethylenically unsaturated monomers are also described in the patent literature.

Aqueous polymer dispersions are moreover obtainable commercially, e.g. with trademarks ACRONAL^{®}, STYRONAL^{®}, BUTOFAN^{®}, STYROFAN^{®}, and KOLLICOAT^{®} from BASF-SE, Ludwigshafen, Germany, VINNOFIL^{®} and VINNAPAS^{®} from Wacker Chemie-GmbH, Burghausen, and RHODIMAX^{®} from Rhodia S.A.

The usual method for free-radical-initiated aqueous emulsion polymerization involves dispersing the ethylenically unsaturated monomers in an aqueous medium, generally with concomitant use of dispersing agents, such as emulsifiers and/or protective colloids, and polymerizing the mixture by using at least one water-soluble free-radical polymerization initiator. In methods frequently used, the residual content of unreacted ethylenically unsaturated monomers in the resultant aqueous polymer dispersions is reduced by chemical and/or physical methods likewise known to the person skilled in the art, polymer solids content is adjusted to a desired value by dilution or concentration, or other conventional additional substances, such as bactericidal, foam-modifying, or viscosity-modifying additives, are added to the aqueous polymer dispersion.

Preferably, the particles of the copolymer contained in the binder polymer have a Z-average particle diameter, as determined by QELS, in the range from 30 to 500 nm, in particular in the range from 40 to 450 nm. The particle size distribution of the copolymer particles contained in the binder polymer may be monomodal or almost monomodal, which means that the distribution function of the particle size has a single maximum and no particular shoulder. The particle size distribution of the copolymer particles contained in the polymer latex may also be polymodal or almost polymodal, which means that the distribution function of the particle size has at least two distinct maxima or at last one maximum and at least a pronounced shoulder.

The copolymer contained in the binder polymer may form a single phase or it may form different phases, if the polymer particles contain different copolymers, which differ with regard to their monomer composition. Preferably, the polymer particles contained in the aqueous polymer latex of the present invention, comprises at least one polymer phase, where the polymer has a glass transition temperature Tg which does not exceed 60°C, in particular is at most 40°C, e.g. in the range from -25 to +40°C, in particular in the range from -20 to +25°C.

The actual glass transition temperature depends from the monomer compositions forming the respective polymer phases (1) and (2), respectively, and a theoretical glass transition temperature can be calculated from the monomer composition used in the emulsion polymerization. The theoretical glass transition temperatures are usually calculated from the monomer composition by the Fox equation: $1 / {Tg}^{t} = {x}_{a} / {Tg}_{a} + {x}_{b} / {Tg}_{b} + … . {x}_{n} / {Tg}_{n} ,$

In this equation xₐ, x_{b}, .... xₙ are the mass fractions of the monomers a, b, .... n and Tgₐ, Tg_{b}, .... Tgₙ are the actual glass transition temperatures in Kelvin of the homopolymers synthesized from only one of the monomers 1, 2, .... n at a time. The Fox equation is described by T. G. Fox in Bull. Am. Phys. Soc. 1956, 1, page 123 and as well as in Ullmann's Encyclopädie der technischen Chemie [Ullmann's Encyclopedia of Industrial Chemistry], vol. 19, p. 18, 4th ed., Verlag Chemie, Weinheim, 1980. The actual Tg values for the homopolymers of most monomers are known and listed, for example, in Ullmann's Encyclopädie der technischen Chemie [Ullmann's Encyclopedia of Industrial Chemistry], 5th ed., vol. A21, p. 169, Verlag Chemie, Weinheim, 1992. Further sources of glass transition temperatures of homopolymers are, for example, J. Brandrup, E. H. Immergut, Polymer Handbook, 1st Ed., J. Wiley, New York 1966, 2nd Ed. J. Wiley, New York 1975, 3rd Ed. J. Wiley, New York 1989 and 4th Ed. J. Wiley, New York 2004.

Usually, the theoretical glass temperature Tg^{t} calculated according to Fox as described herein and the experimentally determined glass transition temperature as described herein are similar or even same and do not deviate from each other by more than 5 K, in particular they deviate not more than 2 K. Accordingly, both the actual and the theoretical glass transition temperatures of the polymer phases (1) and (2) can be adjusted by choosing proper monomers Ma, Mb ... Mn and their mass fractions xₐ, x_{b}, .... xₙ in the monomer composition so to arrive at the desired glass transition temperature Tg(1) and Tg(2), respectively. It is common knowledge for a skilled person to choose the proper amounts of monomers Ma, Mb ... Mn for obtaining a copolymer and/or copolymer phase with the desired glass transition temperature.

The coating compositions of the invention may be formulated as a clear coat or a as a paint. In the latter case, the coating compositions contain, in addition to the polymer binder, at least one inorganic pigment, which imparts a white shade or a color to the coating obtained when using the coating composition for coating substrates.

Pigments for the purposes of the present invention are virtually insoluble, finely dispersed, organic or inorganic colorants as per the definition in German standard specification DIN 55944:2003-11. Examples of pigments are in particular inorganic pigments, such as white pigments like titanium dioxide (C.I. Pigment White 6), but also color pigments, e.g.
- black pigments, such as iron oxide black (C.I. Pigment Black 11), iron manganese black, spinel black (C.I. Pigment Black 27), carbon black (C.I. Pigment Black 7);
- color pigments, such as chromium oxide, chromium oxide hydrate green; chrome green (C.I. Pigment Green 48); cobalt green (C.I. Pigment Green 50); ultramarine green; cobalt blue (C.I. Pigment Blue 28, 36 or 75); ultramarine blue, iron blue (C.I. Pigment Blue 27), manganese blue, ultramarine violet, cobalt violet, manganese violet, iron oxide read (C.I. Pigment Red 101); cadmium sulfoselenide (C.I. Pigment Red 108); molybdate read (C.I. Pigment Red 104); ultramarine red,
- iron oxide brown, mixed brown, spinel- and Corundum phases (C.I. Pigment Brown 24, 29 und 31), chrome orange;
- iron oxide yellow (C.I. Pigment Yellow 42); nickel titanium yellow (C.I. Pigment Yellow 53; C.I. Pigment Yellow 157 und 164); chrome titanium yellow; cadmium sulfide und cadmium zinc sulfide (C.I. Pigment Yellow 37 und 35); Chrome yellow (C.I. Pigment Yellow 34), zinc yellow, alkaline earth metal chromates; Naples yellow; bismuth vanadate (C.I. Pigment Yellow 184);
- Interference pigments, such as metallic effect pigments based on coated metal platelets, pearl luster pigments based on mica platelets coated with metal oxide, and liquid crystal pigments.
- Organic pigments such as phthalocyanine, perylenes, quinacridones, and carbon black.

The coating compositions may also contain one or more fillers. Examples of suitable fillers are aluminosilicates, such as feldspars, silicates, such as kaolin, talc, mica, magnesite, alkaline earth metal carbonates, such as calcium carbonate, for example in the form of calcite or chalk, magnesium carbonate, dolomite, alkaline earth metal sulfates, such as calcium sulfate, silicon dioxide, etc. In the coating compositions of the invention, finely divided fillers are naturally preferred. The fillers may be used in the form of individual components. In practice, however, filler mixtures have been found to be particularly useful, for example calcium carbonate/kaolin, calcium carbonate/talc. Gloss paints generally comprise only small amounts of very finely divided fillers or do not comprise any fillers. Fillers also include matting agents which significantly impair the gloss as desired. Matting agents are generally transparent and may be either organic or inorganic. Examples of flatting agents are inorganic silicates, for example the Syloid^{®} brands from W. R. Grace & Company and the Acematt^{®} brands from Evonik GmbH. Organic flatting agents are obtainable, for example, from BYK-Chemie GmbH under the Ceraflour^{®} brands and the Ceramat^{®} brands, and from Deuteron GmbH under the Deuteron MK^{®} brand.

The proportion of the pigments and fillers in the coating compositions can be described in a manner known per se via the pigment volume concentration (PVC). The PVC describes the ratio of the volume of pigments (VP) and fillers (VF) relative to the total volume, consisting of the volumes of binder (VB), pigments (VP) and fillers (VF) in a dried coating film in percent: PVC [%] = (VP + VF) x 100 / (VP + VF + VB).

If the coating compositions are formulated as a paint, they usually have a pigment volume concentration (PVC) of at least 5%, especially at least 10% and will typically not exceed 90%, in particular 85%. In a preferred group of embodiments, the PVC will not exceed a value of 75%, and preferably in the range from 5 to 60%, more preferrably 5 to 50%. However, the inventive effects of the polymer dispersions are also manifested in varnishes which typically have a pigment/filler content below 5% by weight, based on the varnish, and correspondingly have a PVC below 5%. In yet another group of embodiments, the PVC will be in the range of >75 to 90%,

According to one group of embodiments, the coating compositions of the invention are designed as a paint containing white pigment - that is, they comprise at least one white pigment and optionally one or more fillers. As white pigment they include, in particular, titanium dioxide, preferably in the rutile form, optionally in combination with one or more fillers. With particular preference, the coating compositions of the invention comprise a white pigment, more particularly titanium dioxide, preferably in the rutile form, in combination with one or more fillers, such as chalk, talc or mixtures thereof, for example.

In another preferred group of embodiments, the coating compositions of the invention are designed as a clear-coat or as a wood-stain formulation. In contrast to paints, clear-coats are essentially devoid of pigments and fillers, while wood stains do not contain much fillers, i.e. they have a PVC of below 5%.

Preferably, the coating compositions comprise at least one aqueous polymer latex as defined herein, and can further comprises a rheology modifying agent. Suitable rheology modifying agents include associative thickener polymers and non-associative rheology modifiers. The aqueous liquid composition preferably comprises a thickening agent selected from the group consisting of associative thickeners and optionally a non-associative thickener.

Associative thickener polymers are well known and frequently described in the scientific literature, e.g. by E.J. Schaller et al., "Associative Thickeners" in Handbook of Coating Additives, Vol. 2 (Editor L.J.Calbo), Marcel Decker 192, pp. 105-164, J. Bieleman "PUR-Verdicker" in Additives for Coatings (Editor J. Bielemann), Wiley 2000, pp 50 - 58. NiSAT thickener polymers of the HEUR and HMPE type are also described in the patent literature, such as US 4,079,028, US 4155,892, EP 61822, EP 307775, WO 96/31550, EP 612329, EP 1013264, EP 1541643, EP 1584331, EP 2184304, DE 4137247, DE 102004008015, DE 102004031786, US 2011/0166291 and WO 2012/052508. Apart from that, associative thickener polymers are commercially available. The associative thickener polymers also include non-ionic associative thickeners, so called NiSAT thickeners (non-ionic synthetic associative thickeners), which usually are linear or branched block copolymers having at least one interior hydrophilic moiety, in particular a polyether moiety, especially at least one polyethylene oxide moiety and two or more terminal hydrocarbon groups each having at least 4 carbon atoms, in particular from 4 to 24 carbon atoms, e.g. a linear or branched alkyl radical having 4 to 24 carbon atoms or alkyl substituted phenyl having 7 to 24 carbon atoms. NiSAT thickeners include the hydrophobically modified polyethylene oxide urethane rheology modifiers, also termed HEUR or PUR thickeners, and hydrophobically modified polyethyleneoxides, which are also termed HMPE.

The associative thickener polymers include anionic, acrylate type thickener polymers, so-called HASE polymers (hydrophobically modified polyacrylate thickeners), which are copolymers of acrylic acid and alkyl acrylate monomers, where the alkyl group of the alkyl acrylate may have from 6 to 24 carbon atoms.

The amount of the associative thickener polymer will depend on the desired viscosity profile and is frequently in the range from 0.05 to 2.5% by weight, in particular 0.1 to 2% by weight of thickener, and especially 0.2 to 2% by weight, based on the latex paint.

Suitable non-associative rheology modifiers are in particular cellulose-based thickeners, especially hydroxyethyl cellulose, but also thickeners based on acrylate emulsions (ASE). Amongst the non-associative rheology modifiers preference is given to non-associative cellulose based thickeners.

The total amount of the thickener polymer will depend on the desired viscosity profile and is frequently in the range from 0.05 to 2.5% by weight, in particular 0.1 to 2% by weight of thickener, and especially 0.15 to 1.5% by weight, based on the latex paint.

The aqueous coating compositions of the invention may also comprise customary auxiliaries. The customary auxiliaries will depend from the kind of the coating in a well-known manner and include but are not limited to:
- wetting agents or dispersants,
- filming auxiliaries, also termed coalescents,
- leveling agents,
- UV stabilizers,
- biocides and
- defoamers/de-aerators.
- bases for neutralization (such as NaOH, KOH, NH3, organic amines)

Suitable wetting agents or dispersants are, for example, sodium polyphosphates, potassium polyphosphates or ammonium polyphosphates, alkali metal salts and ammonium salts of acrylic acid copolymers or maleic anhydride copolymers, polyphosphonates, such as sodium 1-hydroxyethane-1,1-diphosphonate, and naphthalenesulfonic salts, especially the sodium salts thereof.

Suitable filming auxiliaries are solvents and plasticizers. Plasticizers, in contrast to solvents, have a low volatility and preferably have a boiling point at 1013 mbar of higher than 250°C, while solvents have a higher volatility than plasticizers and preferably have a boiling point at 1013 mbar of less than 250°C. Suitable filming auxiliaries are, for example, white spirit, pine oil, propylene glycol, ethylene glycol, butyl glycol, butyl glycol acetate, butyl glycol diacetate, butyl diglycol, butylcarbitol, 1-methoxy-2-propanol, 2,2,2-trimethyl-1,3-pentanediol monoisobutyrate (Texanol^{®}) and the glycol ethers and esters, commercially available, for example, from BASF SE under the Solvenon^{®} and Lusolvan^{®} and Loxanol^{®} names, and from Dow under the Dowanol^{®} trade name. The amount is preferably < 5% by weight and more preferably < 1% by weight, based on the overall formulation. Formulation is also possible completely without filming auxiliaries. If the coating compositions contain filming auxiliaries, these are preferably selected from plasticizers. Frequently, the coating compositions do not require any filming auxiliaries. Further suitable auxiliaries and components are e.g. described by J. Bieleman in "Additives for Coatings", Whiley-VCH, Weinheim 2000; by T. C. Patton in "Paint Flow and Pigment Dispersions", 2nd Edition, John Whiley & Sons 1978; and by M. Schwartz and R. Baumstark in "Water based Acrylates for Decorative Coatings", Curt R. Vincentz Verlag, Hanover 2001.

The coating compositions of the invention may also be formulated as a low VOC paint.

### Abbreviations:

- pphm:: parts per 100 monomers
- h:: hour or hours
- RH:: relative humidity
- RT:: room temperature (22-23°C)
- rpm:: revolutions per minute
- nm:: nanometers
- cm:: centimeters
- W:: Watt
- m²:: square meters
- SC:: solids content
- wt%:: % by weight
- rcf:: relative centrifugal force
- US:: ultrasound power setpoint

### Sample preparation and analytics

### C.1 Substrate and application of the paint systems

i) The standard procedure for application for the paints from section examples 1 and 2 are by doctor-blading on pre-weighted glass slides at 25°C and subsequent drying over 7 days. The gap height was 200 µm and the gap width 6 cm, the application was carried out using a doctor-blading machine (Zehntner GmbH, Sissach, Switzerland) to get an even film. After drying, the glass slides were cut to a length of 12 cm and a width of 7 cm and the starting weight noted.
ii) For the flexible roof coating system, the paint from example 3 is applied by doctor-blading on pre-weighted glass slides at 25°C. The gap height was 1000 µm and the application was carried out twice, resulting in a dry film thickness of 1.3-1.4 mm. After drying for 14 days, the glass slides were cut to a length of 12 cm and a width of 7 cm and the starting weight of the coating determined gravimetrically.

### C.2 Artificial UV aging of coated substrates

Artificial aging was performed using an Atlas SUNTEST XXL^{®} conforming to ISO 4892 standardized conditions (Sunlight spectrum, UV intensity of 60 W/m² (max. deviation ± 7.0%) in the wavelength range of 300-400 nm, Black Standard Temperature (BST) of 65°C) with a relative humidity (RH) of 75%. Duplicates of the coated substrates were placed in the device and exposed to the described conditions for 3000 h.

### C.3 Sample preparation for coated glass panels (pristine and aged)

In the first step, the coated substrates made according to the procedures described in section C.1 and C.2 from the coatings in examples 1 to 3 were placed in a glass jar (size 8x18x5 cm), with the coated (and aged) side directly placed onto 6 mL of ultrapure water. The glass jar was then placed onto a laboratory shaker (IKA KS^{®} 130 basic) for 1h at 80 rpm, room temperature. Afterwards, the specimen was removed from the jar and the water was collected for analysis of the dissolved organic carbon (DOC), the collected volume was documented. The specimen was dried at room temperature. In the second step, the same specimen is placed again into the washed and dried glass jar, this time with the uncoated (and unaged) side facing the bottom of the glass jar. The glass jar was fixed in an upright position with an angle of 45°. On the back of the glass jar marker lines with a distance of 15 mm were added. Using an airbrush pistol (Triplex IV^{®}) equipped with a reservoir flask (containing 7 mL of ultrapure water and 0.05 wt.-% Lutensol^{®} AT 18) the liquid was sprayed onto the sample with a pressure of 6 bar. During spraying the marker lines were followed from the bottom of the plate to the top of the plate and to the bottom again. A distance of 9 cm between the nozzle of the airbrush pistol and the plate was kept throughout the spraying procedure. To determine the volume of the water that was sprayed onto the sample, the remaining volume in the reservoir flask was measured and subtracted from the initial volume of 7 mL. The water next to the bottom of the plate contained in the glass jar was collected and its volume was documented. In the third step the specimen was placed again in a glass jar containing 6 mL of ultrapure water and 0.05% Lutensol^{®} AT18 with the coated (and aged) side placed onto the water. The glass jar was placed into a sonication bath (Bandelin Sonorex DigiPlus^{®} DL1028 H) for 1h at a temperature T < 40°C and at the setpoint of 20% US intensity. The specimen was removed from the glass jar, the water was collected and its volume documented. In the final step, the specimen was placed once again in a glass jar containing 6 mL of ultrapure water and 0.05% Lutensol ^{®}AT18 with the coated (and aged) side placed onto the water. The glass jar was placed into a sonication bath (Bandelin Sonorex DigiPlus^{®} DL1028 H) at a setpoint of 100% US intensity. Depending on the coating system to be analyzed, also other intensity levels can be selected. As described above, the sonication bath intensity needs to be chosen in a way that flaking induced by the mechanical treatment only is to be avoided.

### C4. DOC measurements

To determine the concentration of released water-soluble organics, 2 mL of the shaker sample were filtered with a 0.02 µm syringe filter (Whatman^{®} Anotop^{®}) and pH was adjusted to < 3 using 25 wt.-% ortho-phosphoric acid to remove the total inorganic carbon contained. The resulting sample was diluted with ultrapure water with dilution factors of 1:10 and 1:20 and total carbon (sample combusted catalytically on a Pt/Al₂O₃ catalyst in an oxygen stream at ca. 680 °C) was quantified as CO₂ via non-dispersive infrared detection (Shimadzu^{®} TOC-L).

### C5. Particle counter measurements

The released secondary microplastic particles >1 µm were counted with an Abakus^{®} Mobil Fluid laser particle counter (Klotz GmbH) equipped with a laser sensor LDS^{®} 2148(0) (1-120 µm). For this purpose, 0.2 mL of the collected dispersion was filled up to 500 mL with ultrapure water. For sensor protection the sample was filtered with a 190 µm pre-filter (nylon, Rotert). The measurement was performed with 200 mL of the sample. Data evaluation was performed with C.A.R. Lab. Duplicate samples were measured and values for blank measurements were subtracted.

### C6. Pyr-GCMS measurements

The samples for pyr-GCMS measurements were prepared in the following way to wash out/dilute potential dissolved organic carbon (non-particulate): 0.5 mL of the release solution from pristine and aged samples (Airbrush, sonication) were diluted with 3.5 mL ultrapure water. The solution was centrifuged for 1 hour at 20k rpm with a rotor radius of 87.5 mm (Beckman Coulter^{®} ultracentrifuge, SW 41 Ti swinging-bucket rotor), corresponding to 39k ref (ref = 1.12 x radius x (rpm/1000)²), and approximately 3.5-3.7 mL of the supernatant were discarded. The sediment was redispersed and transferred using additional 0.2 mL ultrapure water to a vial for pyr-GCMS measurements. The weight of the final solutions was determined. Depending on the polymer particle density, other centrifugation times or speeds may be used in order to set the lower cut-off for the particle size.

Stock solutions of the references were prepared, by weighing appropriate amounts of the respective paint to an accuracy of 0.01 mg into a Crimp-Vial, finely dispersing the paints in Millipore^{®} water (containing 0.005% Lutensol^{®} AT18) overnight on a roller mixer. Calibration samples were prepared by pipetting appropriate volumes of the stock solutions into a pyrolysis crucible, adding 5 µL of 0.1% trifluoroacetic acid in water and evaporation of the solvent at 120°C for approx. 15 min prior to injection into the pyrolysis system.

Analytical pyrolysis was coupled to capillary gas chromatography mass spectrometry using an analytical pyrolysis system (FrontierLab^{®} EGA/PY-3030D) equipped with an autosampler (FrontierLab^{®} AS-1020E) and coupled to a gas chromatograph (Agilent^{®} 7890B) with mass spectrometric detector (Agilent^{®} 5977A). Balances used were a microbalance (Sartorius^{®} MC 5) and a lab balance (Sartorius^{®} MCA225S). Data acquisition and analysis were done with MassHunter^{®} GC/MS Acquisition and MSD ChemStation^{®}.

### C7. Inductively Coupled Plasma Optical Emission Spectroskopy (ICP-OES) measurements

0.5 mL of the sample (without any further treatment or dilution) was transferred into quartz digestion vessels. The digestion was performed using an automated acid digestion system (BASF construction) and included the following steps:
- Cracking of the sample with a mixture of H₂SO₄ and HNO₃ at approx. 320°C
- Complete digestion of organic remnants with a mixture of H₂SO₄, HNO₃, HClO₄ and H₂O₂ at approx. 160°C
- Removal of excess acids by evaporation almost to dryness
- Dissolution of the digested residue diluted hydrochloric acid by heating to boiling point. The digested solution contained approx. 5% (v/v) HCl

A blank was prepared in an analogous manner. The analytes were determined in the digested solution via inductively coupled plasma optical emission spectrometry (ICP-OES, Thermo Scientific^{®} ICAP 7400 Duo) employing axial view, external calibration and blank subtraction.

### C.8 Characterization of paint formulations:

-pH values of the polymer dispersions were measured according to the standard method
DIN EN 1262:2004-01.
   - Particle Size Distribution of polymer dispersion by DLS The particle diameter of the polymer latex was determined by dynamic light scattering (DLS, also termed quasi-elastic light scattering) of an aqueous polymer dispersion diluted with deionized water to 0.001 to 0.5% by weight at 22°C by means of a HPPS^{®} device from Malvern Instruments, England. What is reported is the cumulant Z average diameter calculated from the measured autocorrelation function (ISO Standard 13321).
   - The minimum film forming temperature (MFFT) was determined in accordance with DIN ISO
2115:2001-04 using a Kofler^{®} heating bank.

### Formulation 1 (for examples 1 and 2)

A paint for exterior wall coating with a PVC of 47% and a solids content of 60% was prepared by mixing the following ingredients in the given order:

| | |
|---|---|
| 195 g | deionized water |
| 3 g | hydroxyethylcellulose thickener (Natrosol^{®} 250 HBR, Ashland) |
| 2 g | aqueous ammonia (25%) |
| 2 g | dispersant 1 (Dispex^{®} AA 4135, BASF) |
| 3 g | dispersant 2 (Calgon^{®} N, 10%, ICL Phosphate Speciality) |
| 2 g | defoamer (Foamaster^{®} MO 2134, BASF) |
| 10 g | propylene glycol |
| 10 g | butyl diglycol |
| 5 g | Texanol^{™} (Ashland) |
| 190 g | TiO₂ pigment (Kronos^{®} 2190, Kronos) |
| 190 g | filler based on calcium carbonate (Omyacarb^{®} 5 GU, Omya) |
| 50 g | talcum filler (Finntalc^{®} M15, Elementis) |
| 320 g | polymer latex (50% by weight) |
| 2 g | defoamer (Foamaster^{®} MO 2134, based on hydrocarbon) |
| 16 g | thickener with Newtonian rheology behavior (Rheovis^{®} PE 1330, BASF) |

The overall solids content of the paint was 58% by weight, the PVC was 47%, paint pH was 8.5.

### Characterization binder 1:

48.5 pphm n-butyl acrylate, 48.7 pphm methyl methacrylate, 1.3 pphm acrylic acid, 1.5 pphm acrylamide, a solids content of 48% and particle size and minimum film formation temperature of 115 nm and 12°C respectively.

### Characterization binder 2:

48 pphm n-butyl acrylate, 52 pphm styrene, 2.5 pphm acrylic acid, 1.7 pphm acrylamide, a solids content of 50% and a particle size of 160nm and minimum film formation temperature of 20°C.

### Formulation 2 (for example 3)

A flexible roof coating with a PVC of 45% and a solids content of 82% was prepared by mixing the following ingredients in the given order:

| | |
|---|---|
| 443 g | Polymer latex (60% by weight) |
| 3 g | Silicone defoamer (Foamstar^{®} SI 2210, BASF) |
| 4 g | Sodium polyacrylate dispersant (Dispex^{®} AA 4135, BASF) |
| 52 g | TiO₂ pigment (Kronos^{®} 2056, Kronos) |
| 191 g | filler based on calcium carbonate 1 (Omyacarb^{®} 2 GU, Omya) |
| 304 g | filler based on calcium carbonate 2 (Omyacarb^{®} 5 GU, Omya) |
| 1 g | Zinc oxide (Zinkoxid Weißsiegel Spezial^{®}, Grillo) |
| 2 g | Associative thickener (Rheovis^{®} PU 1256, BASF) |

The overall solids content of the flexible roof coating was 82%, the PVC was 43%, paint pH was 8.5.

### Characterization binder 3:

90.5 pphm n-butyl acrylate, 8 pphm acrylonitrile, 1.5 pphm methacrylic acid, a solids content of 60%, particle size and minimum film formation temperature of 180 nm and <0°C respectively.

### Example 1

The wet coating weight applied by the doctor-blading on the coated area of 6 cm in width and 12 cm in length is 1.404 g or 195 g/m2. As the liquid paint density is 1.44 g/cm3 and the paint solids content 60%, the dry paint amount corresponds to 0.842 g solid paint.

| Measured parameter \ Sample state | Pristine | | 3000 h Xenotest | |
|---|---|---|---|---|
| Total mass loss after aging before workflow / mg/m² (%) | - | | 1565.48 ± 29.76 (1.34%) | |
| Dissolved organic carbon from shaker / mg/m² | 228.57 ± 7.14 | | 40.71 ± 5.71 | |

| | *Airbrush* | *20% US* | *Airbrush* | *20% US* |
|---|---|---|---|---|
| Visible flaking | No | No | No | No |
| Total polymer mass from pyrolysis GC / mg/m² | < 0.45 | 1.83 ± 0.02 | < 0.45 | 11.16 ± 2.10 |
| Calcium carbonate from ICP-OES / mg/m² | 3.84 ± 0.03 | 13.30 ± 2.70 | 52.04 ± 13.60 | 61.69 ± 6.66 |
| Titanium dioxide from ICP-OES / mg/m² | 0.18 ± 0.03 | 5.28 ± 0.15 | 5.19 ± 0.88 | 97.51 ± 16.64 |
| Aluminum oxide from ICP-OES / mg/m² | 0.54 ± 0.19 | 1.51 ± 0.89 | 1.00 ± 0.07 | 4.17 ± 1.40 |
| Total counts from particle counter / 1/m² | 4191 ± 5 | 59308 ± 2534 | 145652 ± 55328 | 1044985 ± 752996 |

### Example 2

The wet coating weight applied by the doctor-blading on the coated area of 6 cm in width and 12 cm in length is 1.431 g of 199 g/m2. As the liquid paint density is 1.43 g/cm3 and the paint solids content 60%, the dry paint amount corresponds to 0.859 g solid paint.

| | | | | |
|---|---|---|---|---|
| Measured parameter \ Sample state | Pristine | | 3000 h Xenotest | |
| Total mass loss after aging before workflow / mg/m² (%) | - | | 3029.76 ± 89.29 (2.54%) | |
| Dissolved organic carbon from shaker / mg/m² | 300.00 ± 0.00 | | 27.86 ± 3.57 | |

| | *Airbrush* | *20% US* | *Airbrush* | *20% US* |
|---|---|---|---|---|
| Visible flaking | No | minimal | No | Yes |
| Total polymer mass from pyrolysis GC / mg/m² | < 0.45 | 9.43 ± 7.69 | < 0.45 | 152.61 ± 107.79 |
| Calcium carbonate from ICP-OES / mg/m² | 9.81 ± 0.75 | 50.17 ± 22.09 | 21.86 ± 0.78 | 351.66 ± 177.12 |
| Titanium dioxide from ICP-OES / mg/m² | 0.93 ± 0.44 | 13.09 ± 8.15 | 4.28 ± 0.52 | 516.34 ± 229.36 |
| Aluminum oxide from ICP-OES / mg/m² | 6.64 ± 2.94 | 4.57 ± 4.18 | 2.63 ± 1.34 | 21.66 ± 9.47 |
| Total counts from particle counter / 1/m² | 6297 ± 72 | 126505 ± 118714 | 73303 ± 7874 | 3707557 ± 2897549 |

### Example 3

The dry coating weight applied by the dual doctor-blading on the coated area of 7 cm in width and 12 cm in length is 18.86 g of dry coating or 2245 g/m².

| | | | | | | |
|---|---|---|---|---|---|---|
| Measured parameter \ Sample state | Pristine | | | 3000 h Xenotest | | |
| Total mass loss after aging before workflow / mg/m² (%) | - | | | 19309.52 ± 0.00 (1.02%) | | |
| Dissolved organic carbon from shaker / mg/m² | 1678.57 ± 250.00 | | | 292.86 ± 21.43 | | |

| | *Airbrush* | *20% US* | *100% US* | *Airbrush* | *20% US* | *100% US* |
|---|---|---|---|---|---|---|
| Visible flaking | No | No | No | No | No | Yes |
| Total polymer mass from pyrolysis GC / mg/m² | < 0.40 | 0.90 ± 0.07 | 0.60 ± 0.04 | < 0.40 | 0.70 ± 0.10 | 34.51 ± 22.64 |
| Calcium carbonate from ICP-OES / mg/m² | 16.41 ± 0.52 | 32.52 ± 1.45 | 35.40 ± 0.95 | 89.87 ± 24.08 | 126.57 ± 63.45 | 336.37 ± 130.72 |
| Titanium dioxide from ICP-OES / mg/m² | 0.19 ± 0.03 | 0.18 ± 0.01 | 0.12 ± 0.01 | 3.55 ± 0.11 | 11.95 ± 8.27 | 62.68 ± 42.21 |
| Zinc oxide from ICP-OES / mg/m² | 0.16 ± 0.03 | 0.33 ± 0.01 | 0.21 ± 0.01 | 0.59 ± 0.18 | 0.63 ± 0.05 | 1.07 ± 0.27 |
| Total counts from particle counter / 1/m² | 17336 ± 1362 | 30958 ± 22629 | 12405 ± 1044 | 87395 ± 17584 | 41820 ± 291163 | 954009 ± 677273 |

The measured releases after aging under UV-light and humidity and sampling from three different kinds of paints clearly show the variety of different release species from paints and how the paint composition and the sample treatment can influence the amount of release species detected. In the case of low mechanical treatment (Airbrush), the release of particulate species (fillers and polymer particles) is lower than the release of DOC. This was also the case for all pristine samples after each treatment step. However, after environmental aging of the paint samples, the harsher mechanical treatment steps had an influence on the release and could in some cases (example 2 at 3000h, 20% US; example 3 at 3000h, 100% US) also exceed the release of DOC. However, in all cases the release of polymer particle mass represented a minor fraction. To avoid overestimation of microplastic release, the sampling should be stopped as soon as flaking visually occurs. This was the case at 100% ultrasonication for example 3 and example 1, 3000h artificial weathering and at 20% setpoint of ultrasonication power for example 2, 3000h artificial weathering. This shows the importance to adapt the power level of the ultrasonic treatment to the mechanical properties of the investigated coating. In this case it is recommended to either not analyze these samples of the specific treatment step (as done for example 1 at 100% setpoint of ultrasonication power) or to separate the large flakes by sedimentation and analyze the supernatant containing the fine fraction only (as done for example 2, 20% US and example 3, 100% US). If analysis of flakes is of interest, one must determine the particle size distribution of released flakes first before using further mechanical treatments as e.g. milling or further sonication to homogenize the fragments enabling homogeneous subsampling and facilitate the analysis. The sum of the released material (DOC, inorganics, polymer particles) after the procedure (c) from claim 1 is much lower than the mass loss during ageing, showing that most of the organic material is decomposed into low molecular compounds. All coatings were equilibrated for at least 7 days in the same standard atmosphere of 23°C and 50% relative humidity as before the aging procedure, so that differences in residual humidity falsifying the gravimetric weight determination should be ruled out.

## Claims

1. Particle sampling and detection method for polymer comprising coating compositions, based on the following steps,
a. the application and drying of a defined weight of a polymer containing coating formulation on a substrate to form a coating film
b. Ageing of the coating film using UV light and humidity
c. Sampling dissolved organic compounds (DOC), inorganics, micro- and nanoplastics by
c1) absolute mass loss of the coating film under (b) by gravimetrical methods.
c2) dissolved organic compounds with immersion in water
c3) inorganic, micro- and nano plastics with mechanical treatment (e.g. water pressure or ultrasound optionally in the presence of surfactant), and
d. determining the concentration of the released species under c. by quantifying at least one of the following species
d1) dissolved organic carbon,
d2) inorganics, including its fraction in composites,
d3) micro-, nanoplastics, including its fraction in composites,
d4) total number of particles
with analytical detection methods.

2. Particle sampling and detection method according to claim 1, whereby the determination of the concentration of the released species under c. is detected by at least one or a combination of the following analytical detection methods
d1) total organic carbon analyzer,
d2) inductively coupled plasma optical emission spectroscopy (ICP-OES),
d3) pyrolysis GCMS or thermogravimetric analysis or thermoextraction and desorption GCMS,
d4) a particle counting device, a dynamic and static light scattering device or an optical microscopic device.

3. Particle sampling and detection method according to claim 1 or 2, whereby the determination of the concentration of the released species under c. is detected by a combination of the following analytical detection methods
d1) total organic carbon analyzer,
d2) inductively coupled plasma optical emission spectroscopy (ICP-OES),
d3) pyrolysis GCMS
d4) a particle counting device.

4. Particle sampling and detection method according to any of claims 1 to 3, whereby the particles to be detected are below 120 µm of size down to 20 nm particle diameter, determined by dynamic light scattering.

5. Particle sampling and detection method according to any of claims 1 to 4, whereby the ageing under (a) is performed according to ISO 4892.

6. Particle sampling and detection method according to any of claims 1 to 5, whereby the coating composition comprises an aqueous emulsion polymer.

7. Particle sampling and detection method according to any of claims 1 to 6, whereby the coating composition is a paint, adhesive, sealant, or polymer modified plaster or render, insulation coatings, corrosion protection coatings, water-proofing coatings and vibration dampening coatings.

8. Particle sampling and detection method according to any of claims 1 to 7, whereby the coating composition is a paint.

9. A particle detection device, based on at least one or the combination of the following steps,
a. the application and drying of a defined weight of a polymer containing coating formulation on a substrate to form a coating film
b. Ageing of the coating film using UV light and humidity.
c. Sampling dissolved organic compounds (DOC), inorganics, micro- and nanoplastics by
c1) absolute mass loss of the coating film under (b) by gravimetrical methods.
c2) dissolved organic compounds with immersion in water
c3) inorganic, micro- and nano plastics with mechanical treatment (e.g. water pressure or ultrasound optionally in the presence of surfactant), and
d. determine the concentration of the released species under c. is detected by at least one or a combination of the following analytical detection methods d1) total organic carbon analyzer,
d2) inductively coupled plasma optical emission spectroscopy (ICP-OES),
d3) pyrolysis GCMS or thermogravimetric analysis or thermoextraction and desorption GCMS,
d4) a particle counting device, a dynamic and static light scattering device or an optical microscopic device.

10. A particle detection device according to claim 9, which is based on the combination of the following analytical detection methods,
d1) total organic carbon analyzer,
d2) inductively coupled plasma optical emission spectroscopy (ICP-OES),
d3) pyrolysis GCMS
d4) a particle counting device.

11. A Particle sampling and detection device according to any of claims 9 or 10, whereby the ageing under (a) is performed according to ISO 4892.
